# EUROPEAN PATENT APPLICATION

(11) **EP 4 213 157 A1**
(43) Date of publication of application: **19.07.2023**
(21) Application number: 23151748.3
(22) Date of filing: 16.01.2023
(51) Int. Cl.: G16H 40/63, G16H 10/40, G06N 3/02, G06N 20/00

(54) **DEVICE AND METHOD FOR MONITORING RINSING PROCESSES**

(30) Priority: 18.01.2022 LU 102901
(71) Applicant: Stratec SE, 75217 Birkenfeld (DE)
(72) Inventor: KLEINERT, Felix, 75217 Birkenfeld (DE); TAHEDL, Harald, 75217 Birkenfeld (DE); WIENHOLD, Tobias, 75217 Birkenfeld (DE); RECH, Thomas, 75217 Birkenfeld (DE)
(74) Representative: Tegethoff, Sebastian

(57) **Abstract**

The invention relates to a device and a method for monitoring rinsing or flushing processes of parts in automated analyser systems like diagnostic instruments that participate in the handling of liquids.

## Description

### Field of the Invention

The invention relates to a device and a method for monitoring rinsing or flushing processes of parts in automated analyser systems like diagnostic instruments that participate in the handling of liquids.

### Brief description of the related art

Automated analyser systems for use in clinical diagnostics and life sciences are produced by a number of companies. For example, STRATEC^{®} SE, Birkenfeld, Germany, produces a number of devices for specimen handling and detection for use in automated analyser systems and other laboratory instrumentation.

STRATEC designs and manufactures diagnostic instruments with functional subunits, so called modules. These modules take over individual processing steps in the course of the processing of the samples. The functionality required for these processing steps is provided by the firmware of the modules. Usually, data is collected during processing and checked by means of a process control. These process controls serve to analyze the acquired parameters and ensure a correct analysis procedure. If abnormal behavior is detected during process control, the sample currently being processed is discarded if necessary.

Process controls are realized by measuring characteristic properties of the process and applying appropriate thresholds to these measured values. If a measurement exceeds or falls below the defined process limits, it is assumed that abnormal behaviour is present. The process limits are typically defined once during device development.

A central component of these *in vitro* diagnostic devices is the movement of liquids. Among other things, liquid-guided pipettors are used for this purpose. In order to avoid sample carryover due to sticking in the system, these pipettors must be cleaned by rinsing or flushing.

To avoid sample carry-over, a correctly performed rinsing procedure is essential. Factors that can lead to an incorrect rinsing or flushing process include kinking of tubes, idling of system fluid, (partial) clogging of the pipetting needle and bubble formation in the system. *In vitro* diagnostic instruments that cannot control the rinsing process are not able to detect a potential sample carry-over.

Published U.S. patent application US 2021/063361 A1 relates to an automated method of monitoring a state of an analyzer is provided including a mass spectrometer (MS) with an electrospray ionization (ESI) source coupled to a liquid chromatography (LC) stream, including monitoring an electrospray ionization current of the ESI source and identifying a condition of multiple conditions of the analyzer based on the monitored ionization current of the ESI source, one of the conditions being a presence of a dead volume in a liquid chromatography stream of the analyzer downstream of an LC column of the LC stream. The technical solution which is disclosed in this document is based on the determination of an electrospray ionization current of the electrospray ionization which limits the disclosure to applications related to electrospray ionization.

Published European patent application EP 3 671 221 A1 relates to a method for classifying liquid handling procedures, such as pipetting procedures occurring during laboratory automated analyses, said method comprises: receiving measurement data encoding a measurement curve of measurements over time during at least a part of a liquid handling procedure; inputting the measurement data into a neural network; and calculating at least one quality value for the liquid handling procedure with the neural network. The neural network allows to classify whether a liquid handling procedure is normal or abnormal, e.g clotting in the line, presence of air or bubbles, etc.

Published International patent application WO 2021/091755 A1 discloses a method of analyzing a specimen includes detecting a specimen integrity error in the specimen; capturing an image of the specimen; sending the image of the specimen to a customer support center at a remote location; analyzing the image of the specimen at the customer support center; and determining a cause of the specimen integrity error in response to analyzing the image of the specimen.

### Object of the Invention

It is therefore the object of this invention to provide a method

### Summary of the Invention

The present invention provides a method for monitoring the quality of fluid handling in automated analyser systems, comprising the steps of:
- preparing a first set of data, comprising the steps of:
   i. recording a first set of parameters;
   ii. Assigning manually to each parameter curve a value indicating whether the respective parameter curve is judged to be correct or erroneous;
   iii. storing the assessed parameter curves and assigned values in a data base;
- establishing a determination basis, comprising the steps of:
   i. defining multiple features for characterizing a parameter curve;
   ii. executing the features on the recorded parameter curves from the first set of data;
   iii. selecting features from the multiple features reflecting the manually assigned value for each parameter curve from the first set of data;
- monitoring parameter curves in running processes, comprising the steps of:
   i. recording a second set of parameter curves for the same parameters of the first set of parameters during operation of the automated analyser system;
   ii. transferring the data relating to the recorded second set of parameter curves to the data base;
   iii. calculating the quality of the second set of parameter curves by applying the selected features from the multiple features on the second set of parameter curves;
   iv. labelling the second set of pressure curves to be correct or erroneous based on the calculation.

Another aspect of the invention relates to a step that during establishing a determination basis an assessed sum is calculated from a group of features selected from the multiple features so that the assessed sum is in accordance with the manually assigned value.

The Another embodiment may comprise the step that the measured data from the running process are distinguished between four different applications so that for each of the four different applications a determination basis is established.

Another aspect of the invention relates to the determination of x parameter curves with n calculated features for preparing the first set of data, wherein x and n can be any positive integer.

It is further envisaged that the selected features from the multiple features for calculating the assessed sum are changed during monitoring pressure curves in running processes for optimizing the determination basis.

The method according to the present disclosure may further comprise the application of a separate basic machine learning model based on the selected features for each pressure curve.

In another embodiment, the parameter curves are labelled as erroneous or correct based on the output of the applied machine learning models.

Another aspect of the invention relates to manually assigning a value for a correct pressure curve which is 1 and for an erroneous curve which is 0.

The method may further encompass that besides monitoring parameter curves in running processes, the results are controlled by checking whether a different selection of features from the n features provide a more accurate labelling of the pressure curves.

Another aspect of the method relates to the selection of the parameters from the group comprising pressure, flow rate, flow volume or viscosity of the fluid.

Still other aspects, features, and advantages of the present invention are readily apparent from the following detailed description, simply by illustrating preferable embodiments and implementations. The present invention is also capable of other and different embodiments and its several details can be modified in various obvious respects, all without departing from the spirit and scope of the present invention. Accordingly, the drawings and descriptions are to be regarded as illustrative in nature, and not as restrictive. Additional objects and advantages of the invention will be set forth in part in the description which follows and in part will be obvious from the description, or may be learned by practice of the invention.

### Summary of the Figures

The invention will be described based on figures. It will be understood that the embodiments and aspects of the invention described in the figures are only examples and do not limit the protective scope of the claims in any way. The invention is defined by the claims and their equivalents. It will be understood that features of one aspect or embodiment of the invention can be combined with a feature of a different aspect or aspects of other embodiments of the invention, in which:
FIG. 1 shows a scheme depicting the steps of the method according to the invention.
FIG. 2 shows the method steps according to the invention.
FIG. 3 shows a desired pressure curve (left), blocked pipetting needle (middle) and air inclusion in the system (right).
FIG. 4 shows examples of good and bad traces based on measured pressure curves.

### Detailed Description of the Invention and the Figures

The technical problem is solved by the independent claims. The dependent claims cover further specific embodiments of the invention.

As a basis of the invention which will be described in the following, pressure sensors are used for determining, monitoring, and evaluating pipetting processes of fluids qualitatively and quantitatively. The disclosed invention refers to a method for evaluating measured pressure values with a method for obtaining information as to whether a rinsing process for instance has been successfully performed.

The term fluid within the meaning of the present disclosure relates to a liquid, a gas or a mixture thereof, wherein solids can be part of the fluid.

The data from the pressure senor can be used to evaluate whether a pressure deviation from an intended value is present. The pressure curves based on the determined pressure data that have been measured, can be used to detect irregularities relating to
i. Clogged aspiration needles
ii. Insufficient rinsing
iii. Valve leakage
As a conclusion, specific characteristics or features from the pressure curve can be derived to evaluate the quality of the curve and therefore the performance of a rinsing, flushing or washing process.

The core of the invention comprises a method, which evaluates measured values relating to features of a pressure curve recorded during a rinsing, flushing, or washing process and provides information as to whether such a process has been carried out successfully or acceptable regarding the desired quality. Additionally, the method allows to adapt the choice of relevant features from multiple features or a feature library during the lifetime of a device, which may be a subunit or module of an automated analyser system so that the device can be adapted automatically depending from the circumstances during liquid handling.

The goal of a method for process control is to distinguish good from bad rinsing, flushing or washing processes. A classical approach tries to determine characteristic properties or features from measured data, which allow a distinction to be made whether the process was correct or not. In the case of a washing, rinsing or flushing process, this could be, among other things, the maximum pressure over the measuring sequence or the slope of the measured pressure curve over a certain range. This is equivalent to reducing the measurement signal to a smaller number of characteristics or features. Based on the obtained features, threshold values can then be defined for allowing a classification during operation of the device. If not all features, which allow a separation between good and bad cases, are considered during the determination of features, this will lead to a loss of information and possibly increase the number of misclassifications.

One of the challenges is the occurrence of unpredictable faults that may occur during operation, and which comply with the previously defined process limits, as well as correct cases that change over the lifetime of the device and do not comply with the process limits later during operation. Such cases may arise, for example, due to wear and tear of the device and make it necessary to adjust the limit values of the process control. In addition, the measurement data obtained from such *in vitro* diagnostic devices are usually very complex and require therefore a complex and thus time-consuming development to reliably distinguish abnormal process behavior from the desired one. The method according to the present disclosure provides a solution to control such complex processes which may change over time. It is of great interest to be able to automate this development process in the best possible way.

The basis for a method according to the present disclosure is a set of data of multiple pressure curves from washing, flushing or rinsing processes. These pressure curves will be manually inspected and assigned to one of the two classes 'Good' or 'Bad'. The device for performing the method according to the present disclosure is going to distinguish between four flushing processes, designated G1, G2, G3 and G4 in the following. For example, at G1, a flush is carried out periodically to moisten the needle. Furthermore, depending on the processed assay, three further rinsing processes can be distinguished (G2, G3 and G4). These four rinsing processes differ in the rinsing duration, the piston position at the beginning of the rinsing, as well as the movement of the piston during rinsing. The distinction between the four different process has been made in order to achieve a higher quality of the respective process.

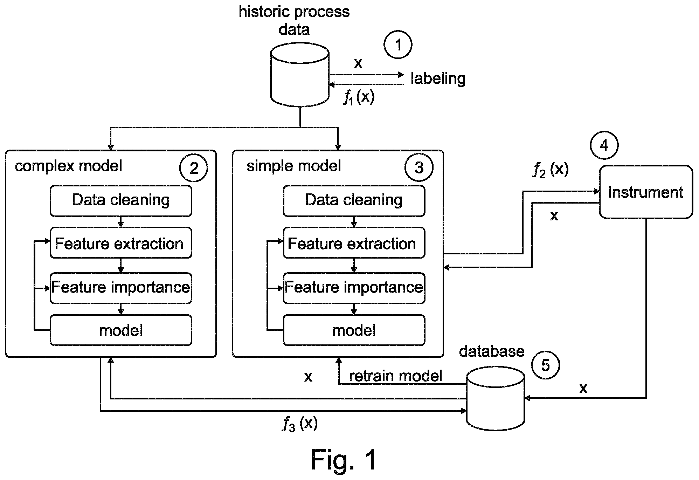

FIG. 1 shows a scheme for describing the interaction of single components of the method according to the invention. The essential steps are numbered from 1 to 5. A complex model 2 and a simple model 3 are obtained based on labelling historical data with values for "good" or "bad" within the meaning of a correct or erroneous process 1. The simple model 3 is executed on the instrument for process control in the firmware and process data from the running operation are stored in a database 5 (locally or in a cloud). The data from this database is labelled with good or bad by the complex model 2 and the simple model 3 may be retrained in appropriate time intervals.

A schematic overview of the embodiment of the method according to the invention is given in FIG. 1. Historical pressure data (1), here exemplary the rinsing process of a water-guided pipettor, are manually divided into the classes of correct and erroneous rinsing process. Domain knowledge about these water-guided modules flows into the manual classification. For example, it is known how air pockets in the system, (partial) blockages of a probe like a pipetting needle or idling of system fluid affects pressure sensor signals. This step must be performed manually and forms the basis for the subsequent automated derivation of classification models. The same or a similar sequence of steps is performed in (2) and (3). The raw data are pre-processed, e.g. interpolation to reconstruct missing measuring points, filtering of the signal for noise suppression. Subsequently, information-bearing characteristics are extracted from the data. Simple non-parameterized statistics, a Fourier transformation, as well as parameterized approaches, e.g. AR models or the adaptation of parameterized functions to the measured data, such as a higher degree polynomial, can be used for this purpose. Based on the extracted characteristics, models are then implemented to classify process data of a washing, flushing or rinsing procedure, e.g. a decision tree or, as in this case, a logistic regression or more complex approaches such as multi-layer neural networks (like convolutional neural networks or recurrent neural networks; RNN) for the "complex" model. The final model from step 3 in FIG. 1 is then implemented in the firmware of an automated analyser system and executed on the instrument for process control during operation (4). FIG. 1 shows in 5 that process data is then transferred to a database (locally, over a network or to a cloud storage service) while the instruments are in operation. Since the number of individual process data is usually very large, manual inspection and labeling of these process data is usually not possible. Therefore, this step is performed by a more complex and actively learning model (2). Here, the strict limitations of computational and memory complexity as in the case of firmware (3) do not apply. With the newly acquired and labelled process data from the running operation, the simpler model from step 3 can then be updated.

This grouping is advantageous because liquid-guided pipettors can usually be in a different state depending on the previously processed assay (varying plunger position) or the flushing time for particularly adherent samples can be selected significantly longer than for less adherent samples. This grouping allows to split a rather complex problem into several easier to solve subproblems. A logistic regression is then performed based on these group-dependent characteristics.

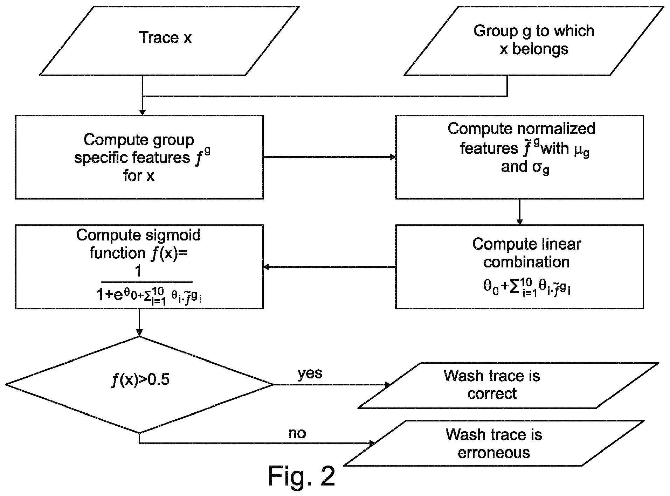

The method steps are shown schematically in FIG. 2 in a flowchart for visualization of the individual steps. For a pressure curve x whose group membership is known, a modeldependent combination of features will be calculated. A list of multiple features that may be taken into account comprises besides further features:
1) The sum over the pressure curve values
2) The sum of all values, that are present in the pressure curve more than once
3) The sum over the absolute value of consecutive changes in the pressure curve
4) The mean over the differences between subsequent pressure curve values
5) The mean over the absolute differences between subsequent pressure curve values
6) Quantiles of the pressure curve
7) Non linearity measures in the pressure curve
8) The absolute energy of the pressure curve which is the sum over the squared values
9) The number of values in the pressure curve that are higher than the mean of the pressure curve
10) The length of the pressure curve
11) The standard deviation of the pressure curve

These calculated characteristics are normalized with the group-dependent mean vector µ_{g} and the group-dependent standard deviation σ_{g}. Then a linear combination of these normalized characteristics f̃^{g} is formed with the regression coefficients θᵢ with I E {0,...,10}. By means of the sigmoid function, an estimate of the class affiliation of the pressure curve is obtained.

From the recorded pressure curve for a known group, specific characteristics are calculated which, depending on the pressure signals of this group, best discriminate good from bad flushing processes. Due to the complexity of these pressure curves it is possible to determine a smaller (and therefore controllable) number of characteristics from a large number of pressure curves of good and bad rinsing processes in a data-driven way. For this purpose, a feature selection can be carried out using optimization methods such as genetic algorithms. In this case, 10 different characteristics per group were determined from a group of 51 preselected characteristics.

The present invention provides a method that allows monitoring of the rinsing or flushing of a pipettor and thus minimizes the risk of a sample carry-over.

Pressure sensors have so far only been used for the detection of liquid surfaces and for the qualitative evaluation of pipetting processes.

The general approach presented by the present invention provides a solution to control complex processes related to handling liquids in automated analyser systems based on determining continuously measurement curves, which may change over time. The described method is related to comparably little effort. The advantages of the described method can be summarized as
1. Possibility to obtain characteristic features of a curve that are relevant for the distinction between good and bad cases.
2. Based on these characteristics, the classification of a curve into good or bad with a linear classification scheme at low computational and memory complexity on an embedded device using the devices' firmware.
3. A second classification scheme that is not subject to the constrained computing and memory capabilities of an embedded device and can therefore extract characteristic properties from the curves to a greater extent and can also perform non-linear separation under certain circumstances. This model is used as an development model running on high performance hardware. The classification results of this model, which is normally of higher quality, can then be used to make estimates about the class affiliation of a curve as a ground truth. This can then be used as additional input to the manually classified data to adapt the linear classification model on the embedded device and improve its quality.

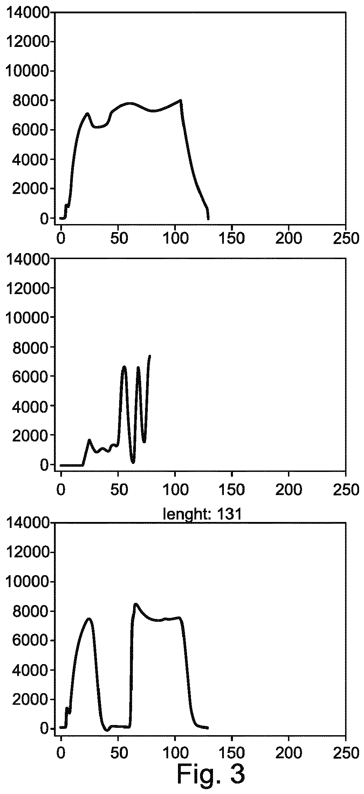

FIG. 3 shows a desired pressure curve (left), blocked pipetting needle (middle) and air inclusion in the system (right).

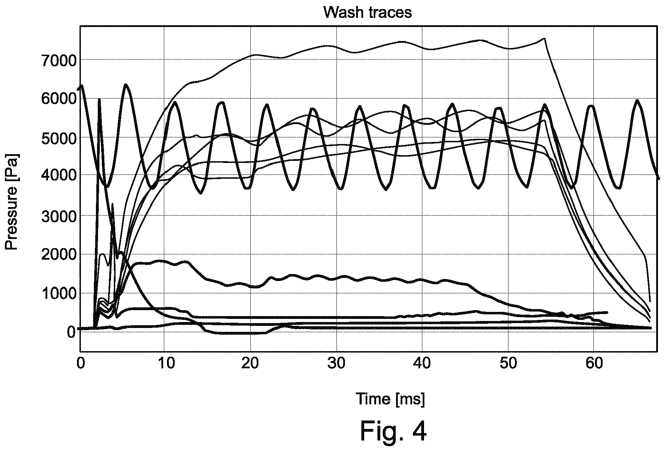

FIG. 4 sshows an example of good and bad traces are compared based on measured pressure curves. The god examples are shown as lines, whereas the bad traces are shown as dotted lines.

It is to be noted that published U.S. patent application US 2021/063361 A1 is based on the measurement of an electrospray ionization current of an electrospray ionization source. Consequently, US 2021/063361 A1 states in paragraph [0021] that monitoring a pressure of the liquid chromatography stream is not helpful as post-column dead volumes might have little or no effect on the pressure curve. US 2021/063361 A1 is based on the measurement of a current, while the present disclosure is based on the determination of properties of fluids like liquids. Thus, the disclosure of US 2021/063361 A1 cannot be used as a basis for a technical solution in an automated analyser system handling fluids.

The foregoing description of the preferred embodiment of the invention has been presented for purposes of illustration and description. It is not intended to be exhaustive or to limit the invention to the precise form disclosed, and modifications and variations are possible in light of the above teachings or may be acquired from practice of the invention. The embodiment was chosen and described in order to explain the principles of the invention and its practical application to enable one skilled in the art to utilize the invention in various embodiments as are suited to the particular use contemplated. It is intended that the scope of the invention be defined by the claims appended hereto, and their equivalents. The entirety of each of the aforementioned documents is incorporated by reference herein.

## Claims

1. A method for monitoring the quality of fluid handling in automated analyser systems, comprising the steps of:
- preparing a first set of data, comprising the steps of:
i. recording a first set of parameters;
ii. Assigning manually to each parameter curve a value indicating whether the respective parameter curve is judged to be correct or erroneous;
iii. storing the assessed parameter curves and assigned values in a data base;
- establishing a determination basis, comprising the steps of:
i. defining multiple features for characterizing a parameter curve;
ii. applying the defined multiple features on the recorded parameter curves from the first set of data;
iii. selecting features from the multiple features reflecting the manually assigned value for each parameter curve from the first set of data;
- monitoring parameter curves in running processes, comprising the steps of:
i. recording a second set of parameter curves for the same parameters of the first set of parameters during operation of the automated analyser system;
ii. transferring the data relating to the recorded second set of parameter curves to the data base;
iii. calculating the quality of the second set of parameter curves by applying the selected features from the multiple features on the second set of parameter curves;
iv. labelling the second set of parameter curves to be correct or erroneous based on the calculation.

2. The method of claim 1, wherein during establishing a determination basis an assessed sum is calculated from a group of features selected from the multiple features so that the assessed sum is in accordance with the manually assigned value.

3. The method of claim 1 or 2, wherein the measured data from the running process are distinguished between four different applications so that for each of the four different applications a determination basis is established.

4. The method of any one of claims 1 to 3, wherein x parameter curves with n calculated features are determined for preparing the first set of data, wherein x and n can be any positive integer.

5. The method of any one of claims 1 to 4, wherein the selected features from the multiple features for calculating the assessed sum are changed during monitoring pressure curves in running processes for optimizing the determination basis.

6. The method of any one of claims 1 to 5, wherein a separate basic machine learning model is applied based on the selected features for each pressure curve.

7. The method of claim 6, wherein based on the output of the applied machine learning models, the parameter curves are labelled as erroneous or correct.

8. The method of claim 1, wherein the manually assigned value for a correct pressure curve is 1 and for an erroneous curve is 0.

9. The method of any one of claims 1 to 8, wherein besides monitoring parameter curves in running processes, the results are controlled by checking whether a different selection of features from the n features provide a more accurate labelling of the pressure curves.

10. The method of any one of claims 1 to 9, wherein the parameters are selected from the group comprising pressure, flow rate, flow volume or viscosity of the fluid.
